Europäisches Patentamt

European Patent Office

Office européen des brevets

(19)

(11) Publication number: **0 032 835**
**B1**

(12)

# EUROPEAN PATENT SPECIFICATION

(45) Date of publication of patent specification: **14.08.85**

(51) Int. Cl.⁴: **A 61 F 2/16**

(21) Application number: **81300222.7**

(22) Date of filing: **19.01.81**

(54) **Intraocular lens.**

(30) Priority: **21.01.80 US 113682**

(43) Date of publication of application:
**29.07.81 Bulletin 81/30**

(45) Publication of the grant of the patent:
**14.08.85 Bulletin 85/33**

(84) Designated Contracting States:
**DE FR GB IT NL**

(56) References cited:
**DE-A-2 717 706**
**GB-A-2 046 099**
**GB-A-2 057 270**
**US-A-4 110 848**

(73) Proprietor: **MINNESOTA MINING AND MANUFACTURING COMPANY**
**3M Center, P.O. Box 33427**
**St. Paul, MN 55133 (US)**

(72) Inventor: **Grinder, William H.**
**499 Alabama St. 114**
**San Francisco California 94110 (US)**

(74) Representative: **Baillie, Iain Cameron et al**
**c/o Ladas & Parry Isartorplatz 5**
**D-8000 München 2 (DE)**

Courier Press, Leamington Spa, England.

## Description

This invention relates to intraocular lenses. Particularly, this invention relates to an intraocular lens for implantation in the posterior chamber of the human eye having at least one resilient spring-like support loop holding the lens in the lens capsule of the posterior chamber of the human eye.

Intraocular lens implantation after cataract surgery has come into common usage because of the improved vision obtained thereby over the alternatives of contact lenses or spectacles. Intraocular lenses have been implanted in both the posterior as well as the anterior chamber of the eye. The first intraocular lens apparently implanted was by Dr. Harold Ridley in 1949. This lens was implanted in the posterior chamber and was implanted following extra-capsular cataract extraction. However, due to a large number of complications, this procedure did not result in wide acceptance of intraocular lens implantation until that which has been obtained in recent years.

As noted, both anterior as well as posterior intraocular lenses have been implanted. The anterior chamber is more readily accessible and has been the position of choice in recent years. Lenses containing loops extending in both the posterior and anterior chambers of the lens and holding the lens at the pupil of the eye have, in the past, been widely used. However, erosion of the iris, due to the contact of the loops and the iris, has caused some difficulty with this type of lens.

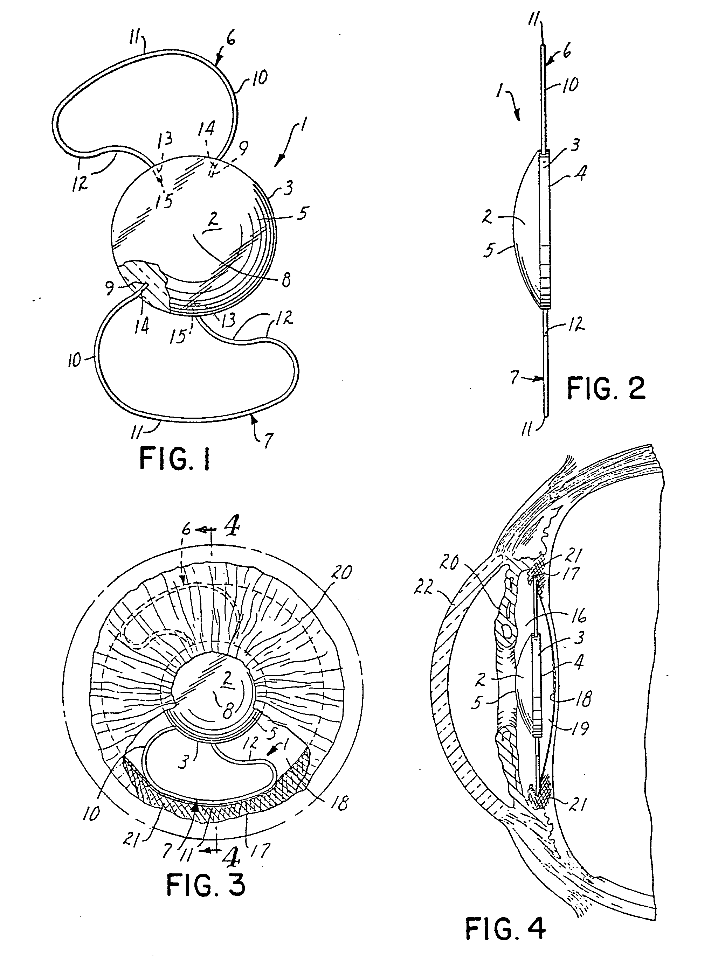

Recently lenses have been utilized for implantation in the posterior chamber of the eye. Examples of such lenses are those disclosed in Kelman U.S. Patent 4,092,743; Richards et al. U.S. Patent 4,014,049, Jensen U.S. Patent 4,110,848, and Shearing U.S. Patent 4,159,546. In the Kelman patent, lenses having three-point contact supporting means are disclosed. These lenses can contain metal wire loops in place of the illustrated solid support members of the patent. The lens can be placed in the posterior chamber of the eye as shown in Figure 3 of Kelman. The lens is held in place by suturing of the lens to the iris.

In the Shearing patent, a posterior chamber lens is disclosed which has two J-shaped elastic support members which are resiliently compressed to allow for the lens to be placed within the posterior chamber of the eye. The lens in Shearing is to have primary ciliary body fixation, thus the J-shaped support members disclosed in the Shearing patent are of sufficient length and resiliency to provide such fixation. However, the J-shaped support members, because of their J shape, do not have substantial resistance to torsional twisting and flexing and it is consequently difficult to accurately and predictably position the lens and its support members in the posterior chamber.

A known intraocular lens for positioning in the eye (see e.g. DE—A—2 717 706) comprises a lens body and first and second support members, wherein said first and second support members extend from the lens body and each of said support members comprises a resilient spring-like support loop extending outwardly from the periphery of said lens body, said support loop being dimensioned and shaped to engage the adjacent portions of the eye with an outward radial force when positioned therein so that the outer contact portion of said resilient loop is deflected inwardly from the normal relaxed position by the adjacent portions of the eye.

While the development of intraocular lenses has proceeded to a level of increased sophistication since the implantation of the Ridley lens, a lens for the posterior chamber, preferably the lens capsule, which is easy to implant yet has stability without suturing has, prior to the present invention, been unknown.

The present invention provides an intraocular lens which is characterized in that said support loop has first and second end portions attached to the lens body at first and second attachment locations, with the portions of each loop that is to engage the eye comprising a curved extent that is longer than the spacing between said first and second end portions and off-set circumferentially of the lens with respect to said first and second end portions and further characterized in that said lens is vertically, horizontally, and rotationally secured in said posterior chamber by the spring action of said support loops in contact with the adjacent portions of the eye without the need for additional sutures, said support loops having sufficient resiliency such that when said support loops are compressed radially said support loops move circumferentially around said lens body and towards said lens body.

Preferred embodiments of the invention are set out in claims 2 to 4.

The lens is designed for easy and essentially automatic and accurate positioning within the posterior chamber, preferably the lens capsule of the eye. The lens is vertically, horizontally and rotationally secured by the spring action of support loops in contact with the interior surface of the outer portion of the posterior chamber of the eye. Preferably, the lens is placed in the lens capsule. There is normally no need for suturing of the support means to any portion of the eye and when the lens is placed in the natural lens capsule there is ordinarily an absence of contact with the ciliary body. If the lens is placed in the lens capsule and there is an attendant absence of contact with the ciliary body, problems which can be caused by contacting the blood carrying vessels of the ciliary body of the eye are reduced. Since the support loop is connected to the lens body on both ends thereof, the lens can be positioned with a minimum possibility of rotational or torsional movement of the lens body in the eye.

The invention will now be described in more detail with reference to the following drawings in which Figure 1 is a front elevation view of the preferred embodiment of the invention. Figure 2 is a side elevation view of the preferred embodiment. Figure 3 is a front elevation view of the preferred embodiment as implanted in the eye, less portions removed for clarity, and Figure 4 is a sectional view of the eye of Figure 3 with the lens in the implanted position.

Referring now to Figures 1 and 2, the preferred embodiment of the intraocular lens of the present invention 1 comprises a lens body 2. The lens body is normally four to six millimeters in diameter and is made by molding or lathing of optical polymeric material such as polymethyl methacrylate. Lens body 2 contains cylindrical surface 3, planar posterior surface 4, and spherical anterior surface 5. The spherical anterior surface 5 is of a desired curvature to give the required optical characteristics which are necessary for the patient in which the lens is implanted.

The lens body is held in place in the eye by means of support loops 6 and 7. These support loops are made of a resilient spring-like material. Normally the material is of a circular configuration and is comprised of polypropylene. Other materials having similar resiliency characteristics and having other cross sectional configurations can be used if they are inert and substantially nonreactive in the human body. The diameter of the loops is normally about 0.15 millimeters. Loops 6 and 7 are in a common plane with the lens body 2, are generally symmetrical with the optical as well as geometric axis 8 of the lens body 2 and are foot-shaped, the ankle portion thereof being attached to lens body 2. In the relaxed condition of the lens 1, as shown in Figure 1, the outer periphery of loops 6 and 7 will generally be approximately twelve millimeters apart if the lens is to be placed in the lens capsule. If the lens is to be placed in the posterior chamber and not in the lens capsule the distance between the outer periphery of loops 6 and 7 will normally be about 13 millimeters. Loops 6 and 7 are of an identical configuration. As shown and preferably, loops 6 and 7 extend beyond lens body 2 in that each loop extends past a line tangent to cylindrical surface 3 of lens body 2 and parallel to a vertical line drawn through geometric axis 8 of lens body 2. Each loop contains a first end portion 9 attached to lens body 2 and leading to a curved leg 10 leading to curved portion 11 which is to contact the outer portion of the posterior chamber of the eye or preferably the equator of the lens capsule of the eye. Curved contact portion 11 is in turn connected to a second leg 12 which forms a smooth S curve back to the lens body 2. Second leg 12 is attached to lens body 2 at second end portion 13. First and second end portions 9 and 13 are respectively attached to the lens body 2 at holes 14 and 15 in lens body 2. The end portions 9 and 13 are radially positioned inside the lens body 2 and are normally bonded in holes 14 and 15 by heat probe, ultrasonic probe, or adhesive in a conventional manner. Lens body 2 and loops 6 and 7 can also be molded unitarily.

The curved contact portion 11 of loops 6 and 7 is designed to have a curve which is substantially equivalent to that of the outer portion of the posterior chamber of the eye or preferably the equator of the lens capsule so that when the loops 6 and 7 are compressed within the posterior chamber or lens capsule the curved portion 11 of loops 6 and 7 follow outer portion of the posterior chamber or the equator of the lens capsule. Since the posterior chamber and lens capsule vary from one patient to the other, often this curve will not track that of the posterior chamber or equator. In some cases, only a portion of the curved contact portion 11 will contact the posterior chamber outer portion or equator of the lens capsule. Additionally, in some cases the lens may be improperly placed in the lens capsule so that one support loop is in the lens capsule while the other is not.

The support loops 6 and 7 contain first and second end portions 9 and 13 with legs 10 and 12 which diverge outwardly from lens body 2 to curved portion 11. The leg 10 is a smooth curve less than 180 degrees while leg 12 is an S configuration having two curved portions oppositely oriented, the first of which and closest to the curved portion 11 is approximately 180 degrees in curvature while the second portion is less than 180 degrees in curvature.

In use the intraocular lens 1 is preferably placed within the eye after the natural lens has been removed from the lens capsule by normal extracapsular cataract removal. Referring to Figures 3 and 4, the central portion 16 of the lens capsule interior surface is removed along with the natural lens. This leaves the equatorial region 17 and posterior wall 18 of the lens capsule in the position shown in Figure 4.

The lens 1 is positioned within the lens capsule 19 by compression of loops 6 and 7. This compression causes loops 6 and 7 to move cylindrically around the lens body 2 and towards the lens body 2. Leg 12 and curved portion 11 of loops 6 and 7 become closer in proximity during the compression and leg 10 is deflected. Support loops 6 and 7 are held against equator 17 of the lens capsule 19. The lens body is positioned so that it does not contact iris 20 of the eye and loops 6 and 7 are preferably positioned so they do not in the normal positioning contact the ciliary body 21 of the eye which contains blood vessels which are subject to rupture. The lens body 2 is held in place by support loops 6 and 7 without the need for additional sutures by means of the outward radial force applied by loops 6 and 7 at curved contact portion 11 against the equator 17 of lens capsule 19. Lens body 2 resides behind iris 20 and cornea 22.

It should be understood that the spirit and scope of the invention is not limited to the preferred embodiment. Modifications of the preferred embodiment will occur to those skilled in the art and are included within the following claims.

## Claims

1. An intraocular lens (1) for positioning in the eye comprising a lens body (2) and first and second support members, wherein said first and second support members extend from the lens body (2) and each of said support members comprises a resilient spring-like support loop (6, 7) extending outwardly from the periphery of said lens body (2), said support loop (6, 7) being dimensioned and shaped to engage the adjacent portions of the eye (17) with an outward radial force when positioned therein so that the outer contact portion (11) of said resilient loop (6, 7) is deflected inwardly from the normal relaxed position by the adjacent portions of the eye (17), characterized in that said support loop (6, 7) has first and second end portions (9 and 13, respectively) attached to the lens body (2) at first and second attachment locations (14 and 15, respectively), with the portions of each loop (6, 7) that is to engage the eye comprising a curved extent (11) that is longer than the spacing between said first and second end portions (9 and 13, respectively) and off-set circumferentially of the lens (2) with respect to said first and second end portions (9 and 13), and further characterized in that said lens (2) is vertically, horizontally, and rotationally secured in said posterior chamber by the spring action of said support loops (6, 7) in contact with the adjacent portions of the eye (17) without the need for additional sutures, said support loops (6, 7) having sufficient resiliency such that when said support loops (6, 7) are compressed radially said support loops (6, 7) move circumferentially around said lens body (2) and towards said lens body (2).

2. A lens according to claim 1 further characterized that said support loop (6, 7) includes a first support leg (10) extending between the first end portion (9) and the outer contact portion (11), said support loop (6, 7) includes a second support leg (12) extending between the second end portion (13) and the outer contact portion (11), and the first and second end portions (9 and 13, respectively) of said support loop (6, 7) extend inwardly from the peripheral edge (3) of the lens body (2).

3. A lens (1) according to claim 2 further characterized in that the first support leg (10) is curved, said outer contact portion (11) is curved, and said second support leg (12) is curved and said first and second support legs (10 and 12, respectively) of said loop (6, 7) outwardly diverge from said first and second end portions (9 and 13 respectively).

4. A lens (1) according to claim 1 further characterized in that said support loops (6, 7) are oppositely disposed on said lens body (2) and oriented circumferentially in a like manner.

## Patentansprüche

1. Intraokulare Linse (1) zum Anordnen im Auge mit einem Linsenkörper (2) und einem ersten und einem zweiten Stützglied, wobei sich das erste und das zweite Stützglied von dem Linsenkörper (2) weg erstreckt und jedes der Stützglieder eine elastische, federartige Stützschlaufe (6, 7) besitzt, die sich vom Umfang des Linsenkörpers (2) auswärts erstreckt und so bemessen und ausgebildet ist, daß sie an den benachbarten Teilen des Auges (17) mit einer radial auswärtsgerichteten Kraft angreift, wenn die Linse im Auge derart angeordnet ist, daß der äußere Anlageteil (11) der elastischen Schlaufe (6, 7) von den in ihrer normalen, entspannten Stellung befindlichen, benachbarten Teilen des Auges (17) einwärts ausgelenkt wird, dadurch gekennzeichnet, daß die Stützschlaufe (6, 7) einen ersten und einen zweiten Endteil (9, 13) besitzt, die an dem Linsenkörper (2) an einer ersten bzw. zweiten Befestigungsstelle (14, 15) angebracht sind, daß die an dem Auge angreifenden Teile jeder Schlaufe (6, 7) einen gekrümmten Bereich (11) besitzen, dessen Länge größer ist als der Abstand zwischen dem ersten und dem zweiten Endteil (9, 13) und der in der Umfangsrichtung der Linse (2) gegenüber dem ersten und dem zweiten Endteil (9, 13) versetzt ist, ferner dadurch gekennzeichnet, daß durch die Federwirkung der an den benachbarten Teilen des Auges (17) angreifenden Stützschlaufen (6, 7) die Linse in der hinteren Augenkammer gegen eine vertikale, horizontale und Drehbewegung festgelegt ist, ohne daß zusätzliche Nähte erforderlich sind, und daß die Stützschlaufen (6, 7) so elastisch sind, daß sie bei ihrem radialen Zusammendrücken sich in der Umfangsrichtung des Linsenkörpers (2) und zu diesem hin bewegen.

2. Linse nach Anspruch 1, dadurch gekennzeichnet, daß die Stützschlaufe (6, 7) einen ersten Stützschenkel (10) besitzt, der sich zwischen dem ersten Endteil (9) und dem äußeren Anlageteil (11) erstreckt, daß die Stützschlaufe (6, 7) einen zweiten Stützschenkel (12) besitzt, der sich zwischen dem zweiten Endteil (13) und dem äußeren Anlageteil (11) erstreckt, und daß sich der erste und der zweite Endteil (9, 13) der Stützschlaufe (6, 7) vom Umfangsrand (3) des Linsenkörpers (2) einwärts erstrecken.

3. Linse (1) nach Anspruch 2, dadurch gekennzeichnet, daß der erste Stützschenkel (10), der äußere anlageteil (11) und der zweite Stützschenkel (12) gekrümmt sind und der erste und der zweite Stützschenkel (10, 12) der Schlaufe (6, 7) von dem ersten und dem zweiten Endteil (9, 13) weg divergieren.

4. Linse (1) nach Anspruch 1, dadurch gekennzeichnet, daß die Stützschlaufen (6, 7) auf entgegengesetzten Seiten des Linsenkörpers (2) angeordnet sind und sich in der Umfangsrichtung in demselben Sinn erstrecken.

## Revendications

1. Lentille intra-oculaire (1) pour mise en place dans l'oeil, comprenant un corps de lentille (2) et des premier et deuxième supports, dans laquelle les premier et deuxième supports partent du corps de lentille (2) et chacun des supports comprend une boucle élastique de maintien (6, 7) semblable à un ressort et dirigée vers l'extérieur à partir de la périphérie du corps de lentille (2), cette boucle de maintien (6, 7) ayant une dimension et une configuration telles qu'elle vient en contact avec les parties adjacentes de l'oeil (17) avec une force radiale vers l'extérieur lorsqu'elle est en place dans l'oeil, de sorte que la partie extérieure de contact (11) de la boucle élastique (6, 7) est fléchie vers l'intérieur, à partir de la position normale de repos, par les parties adjacentes de l'oeil (17), caractérisée en ce que la boucle de maintien (6, 7) comporte des première et deuxième parties d'extrémité (9 et 13, respectivement) attachées au corps de lentille (2) à un premier et un deuxième endroits de fixation (14 et 15, respectivement), les parties de chaque boucle (6, 7) qui doivent venir en contact avec l'oeil comprenant une région courbe (11) plus longue que la distance entre les première et deuxième parties d'extrémité (9 et 13, respectivement) et décalée dans la direction circonférentielle de la lentille (2) par rapport aux première et deuxième parties d'extrémité (9 et 13), et caractérisée en outre en ce que la lentille (2) est fixée verticalement, horizontalement et en rotation dans la chambre postérieure, par l'action élastique des boucles de maintien (6, 7) en contact avec les parties adjacentes de l'oeil (17), sans nécessiter de sutures additionnelles, les boucles de maintien (6, 7) ayant une élasticité suffisante pour que, lorsque les boucles de maintien (6, 7) sont comprimées radialement, les boucles de maintien (6, 7) se déplacent circonférentiellement autour du corps de lentille (2) et vers ce corps de lentille (2).

2. Lentille suivant la revendication 1, caractérisée en ce que la boucle de maintien (6, 7) comprend une première branche de maintien (10) s'étendant entre la première partie d'extrémité (9) et la partie extérieure de contact (11), la boucle de maintien (6, 7) comprend une deuxième branche de maintien (12) s'étendant entre la deuxième partie d'extrémité (13) et la partie extérieure de contact (11), et les première et deuxième parties d'extrémité (9 et 13, respectivement) de la boucle de maintien (6, 7) s'étendent vers l'intérieur à partir du bord périphérique (3) du corps de lentille (2).

3. Lentille (1) suivant la revendication 2, caractérisée en ce que la première branche de maintien (10) est courbe, la partie extérieure de contact (11) est courbe, et la deuxième branche de maintien (12) est courbe, et les première et deuxième branches de maintien (10 et 12, respectivement) de la boucle (6, 7) divergent vers l'extérieur à partir des première et deuxièmes parties d'extrémité (9 et 13, respectivement).

4. Lentille (1) suivant la revendication 1, caractérisée en ce que les boucles de maintien (6, 7) sont disposées en opposition sur le corps de lentille (2) et sont orientées circonférentiellement de façon identique.

0 032 835

FIG. 1

FIG. 2

FIG. 3

FIG. 4

1